# EUROPEAN PATENT APPLICATION

(11) **EP 1 525 866 A1**
(43) Date of publication of application: **27.04.2005**
(21) Application number: 03741558.5
(22) Date of filing: 24.07.2003
(51) Int. Cl.: A61F 13/511

(54) **TOP SHEET OF HYGROSCOPIC ARTICLE, HYGROSOCOPIC ARTICLE USING THE TOP SHEET, AND SELECTION AND EVALUATION METHOD FOR TOP SHEET**

(30) Priority: 25.07.2002 JP 2002216077
(71) Applicant: Uni-Charm Corporation, Shikokuchuo-shi, Ehime 799-0111 (JP)
(72) Inventor: KURATA, Yuri c/o Technical Center, Uni-Charm Corp., Mitoyo-gun, Kagawa 769-1602 (JP); SATO, Junko c/o Technical Center, Uni-Charm Corp., Mitoyo-gun, Kagawa 769-1602 (JP); KURATA, Nobuhiro c/o Technical Ctr Uni-Charm Corp., Mitoyo-gun, Kagawa 769-1602 (JP); YAMADA, Yozo c/o Technical Center, Uni-Charm Corp., Mitoyo-gun, Kagawa 769-1602 (JP); HISANAKA, Takayuki c/o Technical Ctr. Uni-CharmC., Mitoyo-gun, Kagawa 769-1602 (JP); ISHIMARU, Sonoko c/o Toyobo Co., Ltd. Research Ctr, Ohtsu-shi, Shiga 520-0243 (JP); NISHINAKA, Hisao c/o Toyobo Co., Ltd., Osaka-shi, Osaka 530-8230 (JP)
(74) Representative: Purdy, Hugh Barry
(86) International application number: PCT/JP2003/009379
(87) International publication number: WO 2004/010917

(57) **Abstract**

The topsheet of a absorbent article improved dry feeling, and an evaluation/selection method for a topsheet improved in dry feeling. The liquid-permeable topsheet of a absorbent article, **characterized in that** a contact cool feeling during a wet condition as measured in a maximum heat transfer quantity (q-max value) is less than 1.1 kw/m² on the side contacting the skin oft wearer of the topsheet, and a q-max value on the side contacting the absorbent article is larger than a q-max value on the side contacting the skin of a wearer with the difference of at least 0.5 kw/cm². An evaluation/selection method for the topsheet of a absorbent article excellent in dry feeling using a q-max value during a wet condition as an index.

## Description

### Technical Field

The present invention relates to a topsheet of an absorbent article, the absorbent article using the topsheet, and a method for selecting/evaluating the same. More particularly, the present invention relates to an improvement of dry feeling on a topsheet used for an absorbent article such as a disposable diaper and sanitary napkin.

### Prior Art

An absorbent article such as a disposable diaper and sanitary napkin typically comprises an absorbent core made of a highly absorbable material, a liquid permeable topsheet at the side in contact with skin of the wearer and a liquid impermeable backsheet at the opposite side thereof, which sandwich the core.

In such absorbent articles, first, excellent absorption performance of the absorbent core such as large absorbed amount, high absorption speed, and less backflow amount is required.

Furthermore, to improve a fit of the wearer, the performance of the topsheet thereof plays an important role.

However, it is an actual situation that numerous ingenuities for improving the absorption performance of the absorbent core have been made up to now, whereas only few ingenuities for improving the performance of the topsheet have been made.

As the topsheet of the absorbent article, non-woven fabrics of polyolefin, polyester or the like have been used, and in order to improve water permeability thereof, methods of treating these non-woven fabric surfaces with surfactants and/or water-repellents are known (see JP 54-153872 A and JP 61-15192 A).

Also, recently, an invention aimed to get a dry feeling of the topsheet by evaluating not only the water permeability of the topsheet but also a slantingly flowing time thereof to make the absorption speed fast and make the backflow amount small in the topsheet of the absorbent article has been disclosed (JP 2000-333991 A). In the invention, the dry feeling of topsheet is tried to make favorable specifically by adjusting the slantingly flowing time at the side in contact with the skin of the topsheet to 0 to 2.5 seconds and adjusting the slantingly flowing time at the other side to 2.5 to 10 seconds to make the absorption speed fast and make the backflow amount small. As a method for controlling the slantingly flowing time, well-balanced coating of a hydrophilizing agent is given.

However, studies of the present inventors have revealed that the dry feeling sensed when the absorbent article is worn depends on a time from excretion of body fluids to passing through the topsheet of the absorbent article and an amount of the liquid present between the skin and the absorbent article after the body fluids have passed through the topsheet. In the prior art such as JP 2000-333991 A disclosed above, the water permeability and the absorption speed which correspond to the time until passing through the topsheet of the absorbent article have been improved, but the liquid amount after passing through the topsheet has not been improved.

An object of the present invention is to solve the above problems of the prior art.

That is, an object of the present invention is to provide a topsheet of an absorbent article with improved dry feeling and an absorbent article used the topsheet. Furthermore, another object of the present invention is to provide a method of evaluating/selecting a topsheet with improved dry feeling and an absorbent article used this.

### Disclosure of the Invention

As a result of extensive studies to solve the above problems, the inventors of the present invention have conceived an idea that a dry feeling of a topsheet can be evaluated if a contact warm/cool feeling sensed when human skin contacts with an object can be used as an index, and accomplished the present invention.

Furthermore, the inventors have selected and/or evaluated the topsheet with favorable dry feeling by introducing a maximum heat transfer quantity (q-max value) regarding heat transfer characteristics as an objective index for evaluating the contact warm/cool feeling.

That is, the present invention relates to a liquid permeable topsheet of an absorbent article characterized in that a maximum heat transfer quantity (q-max value) during a wet condition measured as a contact warm/cool feeling is 1.1 kw/m² or less on a face at a side in contact with skin of a wearer of the topsheet and a q-max value on a face at a side in contact with an absorption body is 0.5 kw/m² or more larger than the q-max value on the face at the side in contact with the skin of the wearer.

In the present invention, by making a q-max value of the topsheet during a wet condition on a face at the side in contact with the skin smaller than that on a face at the side in contact with an absorption body and setting a difference between the q-max values of both, it was possible to eliminate a wet feeling at the side in contact with the skin, enable smooth migration of body fluid, and consequently improve the dry feeling of the topsheet. The difference of q-max values referred to herein includes step-like difference in value in a thickness direction of the topsheet, difference having a certain continuous gradient, or difference between the front side and the rear side.

The aforementioned topsheet is preferably used for an absorbent article including: the topsheet; a liquid impermeable backsheet; and an absorbent core disposed between the topsheet and the backsheet.

Also, the present invention relates to a method for selecting and/or evaluating a topsheet of an absorbent article with a favorable dry feeling by using a maximum heat transfer quantity (q-max value) during a wet condition measured as a contact warm/cool feeling as an index.

More specifically, the present invention relates to a method for selecting and/or evaluating a topsheet of an absorbent article with a favorable dry feeling by using as a criterion that a q-max value during a wet condition is 1.1 kw/m² or less on a face at a side in contact with skin of a wearer of the topsheet and a q-max value on a face at a side in contact with an absorption body is 0.5 kw/m² or more larger than the q-max value on the face at the side in contact with the skin of the wearer.

By using the maximum heat transfer quality (q-max value) during the wet condition as the index, it is possible to objectively evaluate the dry feeling of the topsheet, and it also becomes possible to select and/or evaluate the topsheet which meets a fit of wearers in the production site of the topsheet.

### Brief Description of the Drawing

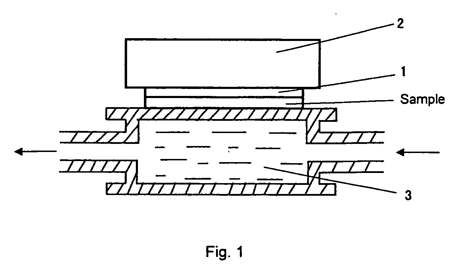

Fig. 1 is a schematic view of a q-max value measurement apparatus according to the present invention.

Reference numeral 1 denotes a pure copper plate; 2, a T-BOX; and 3, a Water box.

### Best Mode for carrying out the Invention

The present invention is described in detail below, but the present invention is not limited thereto.

The absorbent articles of the present invention include paper diapers, sanitary napkins, vaginal discharge liners, and breast milk pads, and additionally the present invention can also be applied to shorts for incontinence.

The present invention is characterized in that a q-max value is used for evaluating a dry feeling of a topsheet of the absorbent article.

It is described that heat transfer characteristics, the q-max value (maximum heat transfer quantity) is used as one evaluation criterion of texture of diapers in "THERMO-PHYSICAL PROPERTIES OF A NON-WOVEN FABRIC COMPOSITES FOR WATER-ABSORBING HYGIENE PRODUCTS" in the Proceedings of the 29th Textile Research Symposium. It is said that texture values such as softness and slipping property, and transfer characteristics of water and heat of the diaper itself are deeply associated with amenity that people feel, and in this paper, a relation of the q-max value with a moisture percentage and a water content of the absorbent article is represented by a regression formula. That is, a relation of the heat transfer characteristics such as q-max values with the absorption performance such as liquid backflow quantity is surveyed by dripping water onto a diaper loaded with 4.5 kPa load which corresponds to an average body weight of a one-year old baby. However, this research paper relates to properties of an absorbent core of the diaper, but does not relate to properties of a topsheet, nor to a dry feeling.

The q-max value is a measurement value adapted to simulate heat transfer, and is related to a contact warm/cool feeling sensed when an article such as absorbent article is brought into contact with human skin.

In the present invention, the q-max value (kw/m²) is the maximum heat transfer quantity measured using a precise rapid heat physical property measurement apparatus. The precise rapid heat physical property measurement apparatus, for example, includes THERMO LABO II (KES-F7) manufactured by Kato Tech Co., Ltd. A q value is the value obtained by accumulating heat in a pure copper plate applied on a heat insulating material, and immediately after this contacts with a sample surface, measuring heat flux that the accumulated heat transfers to the sample at the low temperature side. A heat flux signal reaches a peak about 0.2 seconds after the contact, and this peak value is defined as the q-max value. The q-max value is related to a warm/cool feeling sensed when the human skin contacts the sample object.

Specifically, the q-max value of the topsheet during the wet condition is measured by the following method. The measurement is performed in a temperature and humidity controlled room where the room temperature and humidity are set at 20°C and 65% RH, respectively.

Fig. 1 is a schematic view of a measurement apparatus of the q-max value. The temperature control system of KES-F7 comprises a pure copper plate (1) (area: 9 cm², mass: 9.79 g) applied onto a heat insulating material, T-BOX (2) of a heat source which gives a temperature difference to a sample, and Water box (3) which has a constant temperature plate for retaining the sample at ambient temperature.
The pure copper plate (1) is a heat storing plate and has a built-in precise temperature sensor. In Water box (3), heat capacity is made infinite by running water with a fixed temperature inside thereof.

A sample (topsheet) of which q-max is measured is left in the room with constant temperature and humidity (room temperature at 20°C and humidity at 65% RH) for one or more weeks in advance. The temperature of the pure copper plate (1) having an area of 25 cm² is set at 30°C, and the temperature of Water box (3) is set at 20°C. The topsheet (6.5 cm x 6.5 cm) of the absorbent article which is a sample is overlaid on a dummy absorption body where 10 sheets of filter papers (corresponding to the No.2 grade filter paper in JIS P3801) having a size of 10 cm x 10 cm are overlapped. The topsheet and the filter papers in this state are placed on Water box (3), and 0.085 g/cm² of saline at 20°C (0.9% NaCl) is evenly dripped on the topsheet. After dripping, they are left for one minute. When left for one minute, commonly the saline permeates through completely. Thereafter, the pure copper plate (1) set at 30°C is quickly moved onto the topsheet on Water box (3), and the maximum heat transfer quantity, i.e. q-max value (kw/m²), is read out. The q-max value is represented as a function of time by differentiating a heat transfer quantity q transferring from the pure copper plate (1) which is a heat storing plate to the sample with a copper plate temperature T. The larger the value of the maximum heat transfer quantity (q-max value), the larger a cold feeling when contacted (see Journal of Textile Machinery Society of Japan, Vol.37, No.8 (1984), pp130-141).

In the present invention, the q-max value during the wet condition is used as an index. This value "during the wet condition" refers to the measurement value after the saline is dripped on and permeates through the dummy absorption body having the topsheet as mentioned above.

When this q-max value is measured, if measured by placing the topsheet on Water box with the side in contact with the skin of the topsheet up, the q-max value at the side in contact with the skin of the topsheet can be measured. Conversely, if measured by placing the topsheet on Water box with the side in contact with the absorption body of the topsheet up, the q-max value at the side in contact with the absorption body of the topsheet can be measured.

In the present invention, the q-max value of the topsheet with favorable dry feeling is 1.1 kw/m² or less at the side in contact with the skin of the wearer of the topsheet, and the q-max value at the side in contact with the absorption body is larger than the q-max value at the side in contact with the skin of the wearer, and the difference is 0.5 kw/m² or more. If the q-max value at the side in contact with the skin exceeds 1.1 kw/m², the wet feeling occurs when the skin touches the topsheet. Also, by bringing the difference of 0.5 kw/m² or more between the q-max values at the side in contact with the skin and the side in contact with the absorption body, migration of a liquid from the face in contact with the skin to the face of absorption body side is made smooth.

The q-max value is as mentioned above and therefore, it is the value which is associated with quantities of water which remains in the topsheet and/or the absorbent core, and the q-max value tends to decrease when the remaining water quantity is small. Thus, it is presumed that the remaining water quantity at the skin side can be reduced by making the q-max value at the skin side small and the q-max value at the absorption body side large.

Non-woven fabrics used as the topsheet in the present invention comprises, for example, 1 to 5 d synthetic fiber such as polyolefin-based synthetic fiber, polyester-based synthetic fiber or the like, semi-synthetic fiber such as rayon or the like, or natural fiber such as pulp, cotton or the like.

As methods of manufacturing the non-woven fabric, there are dry processes and wet processes such as thermal bond, spun bond, air raid, chemical bond, spun lace or the like. Generally, the dry process is frequently used for the topsheet of the absorbent article, and particularly the thermal bond non-woven fabric is preferable due to well-balanced strength and texture.

The topsheet is not limited to the non-woven fabrics and may be a perforated film. As the perforated film, it is possible to use liquid permeable perforated films obtained by extruding the film made up of a thermoplastic resin, and subsequently perforating the resultant by hot needle, emboss, hot wind, and the like. As the thermoplastic resin which forms the perforated film, it is possible to use polyethylene, polypropylene and the like alone or in mixture thereof. Also, as the perforated film, it is possible to use those where the same types or different types are laminated in combination.

Furthermore, the topsheet of the absorbent article is necessary to satisfy requests of water resistance and air permeability. Regarding the water resistance, at least 0 to 300 mmH₂O of the water resistance based on JIS L1092 "Testing methods for water resistance of textiles" water resistance test method A (low water pressure method) is required. Regarding air permeability (permeability of vapor phase water), at least 5 to 700 cm³/cm²/second of an air permeability degree based on JIS L1096 "Testing methods for general textile products" air permeability method A (fragile method) is required.

When the topsheet is made up of the non-woven fabric, water easily remains at intersecting points of fibers, and thus if fiber density is high, a remaining water content becomes large, and the q-max value becomes large.

Therefore, it is also possible to manufacture the topsheet of the present invention by giving gradient to a fiber weight per unit area, i.e., a fiber density. Conventionally, there have been the topsheets with the density gradient, but the density in the face at the side in contact with the skin is higher, or the density gradient is less than that in the present invention. No topsheet having the density gradient such that the difference of the q-max values on the face at the side in contact with the skin and face of the topsheet at the side in contact with the absorption body is 0.5 kw/m² or more has been provided.

Also, by giving the gradient to fineness of the face of the topsheet at the side in contact with the skin and the face at the absorption body side, it is also possible to reduce the q-max value on the face in contact with the skin and increase the difference from the opposite side face.

The absorbent article using the topsheet of the present invention comprises the topsheet of the present invention, the liquid impermeable backsheet, and the absorbent core disposed between both the sheets, which is made up of highly absorbable materials. It is preferred that the backsheet is a liquid impermeable polyethylene sheet and the absorbent core is the absorption body or the absorbent sheet made up of absorbent fibers of flocculent pulp, rayon or the like including highly absorbable resins.

Hereinafter, the present invention is described in more detail by Example and Comparative Examples, but the present invention is not limited thereto.

### Example and Comparative Examples

A thermal bond non-woven fabric was manufactured by an air through bonding method to make a topsheet. Core-in-sheath fibers with an average fineness of 2.2 d and an average fiber length of 4.4 mm with core/sheath of polyethylene terephthalate 50/polyethylene 50
were used for a skin face side, and
core-in-sheath fibers with an average fineness of 1.9 d and an average fiber length of 4.4 mm with core/sheath of polyethylene terephthalate 50/polyethylene 50 were used for an absorption body side.
Both were laminated to become 11 g/m² of weight per unit area (Metsuke) at the skin face side and 11 g/m² of weight per unit area at the absorption body side.
An air through fusion temperature was 140°C.
A thickness measured according to JIS L1906 was 0.55 mm.
The measurement of the thickness was performed
under the condition of 0.3 Pa (3 gf/cm²) of load and 15 cm² of gauge head area by changing the corresponding values specified in JIS L1906.

Similarly, the non-woven fabrics with properties shown in Table 1 were manufactured to make Comparative Examples 1 to 5. Comparative Example 5 is the non-woven fabric obtained by bonding continuous fibers with fineness of 2.0 d by point bond.

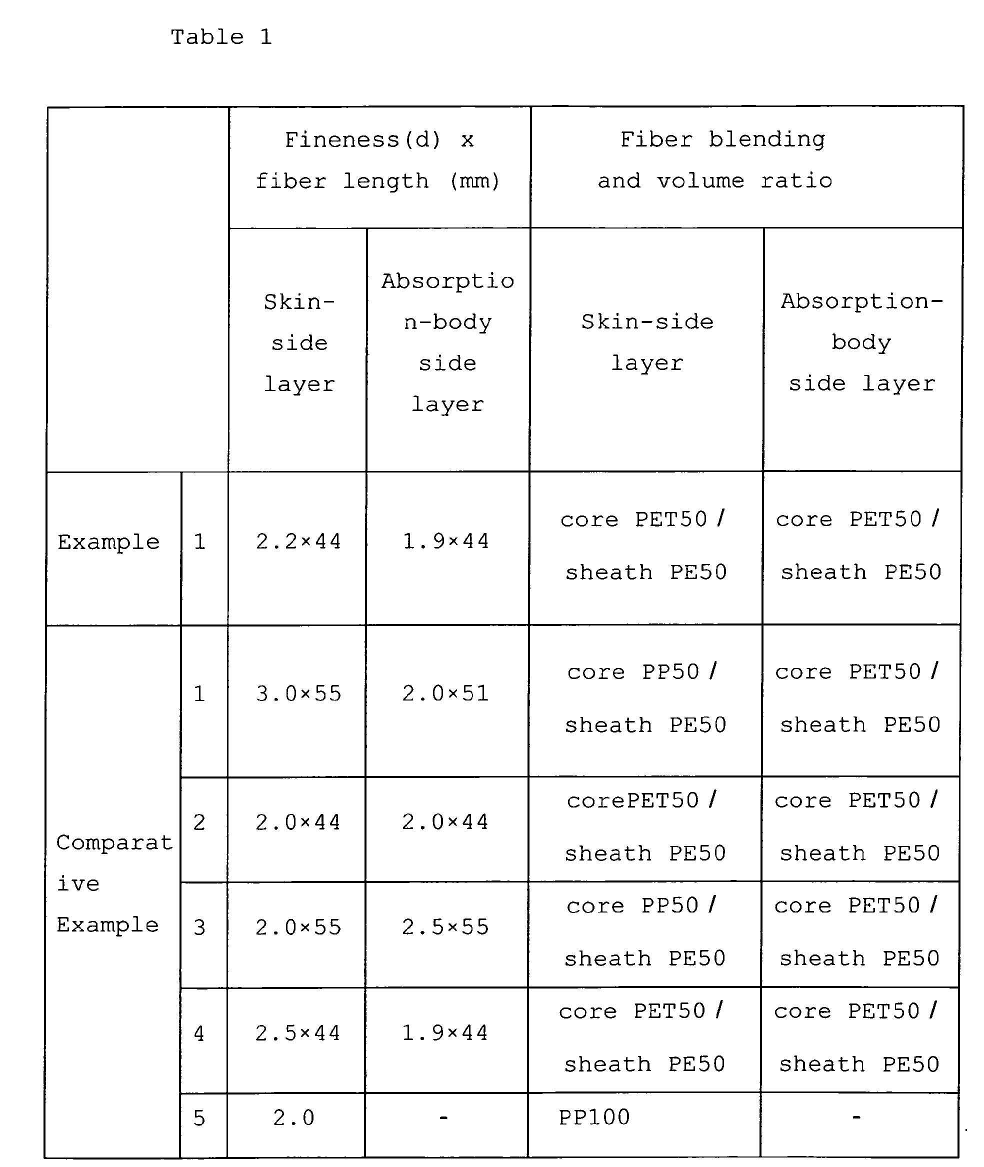

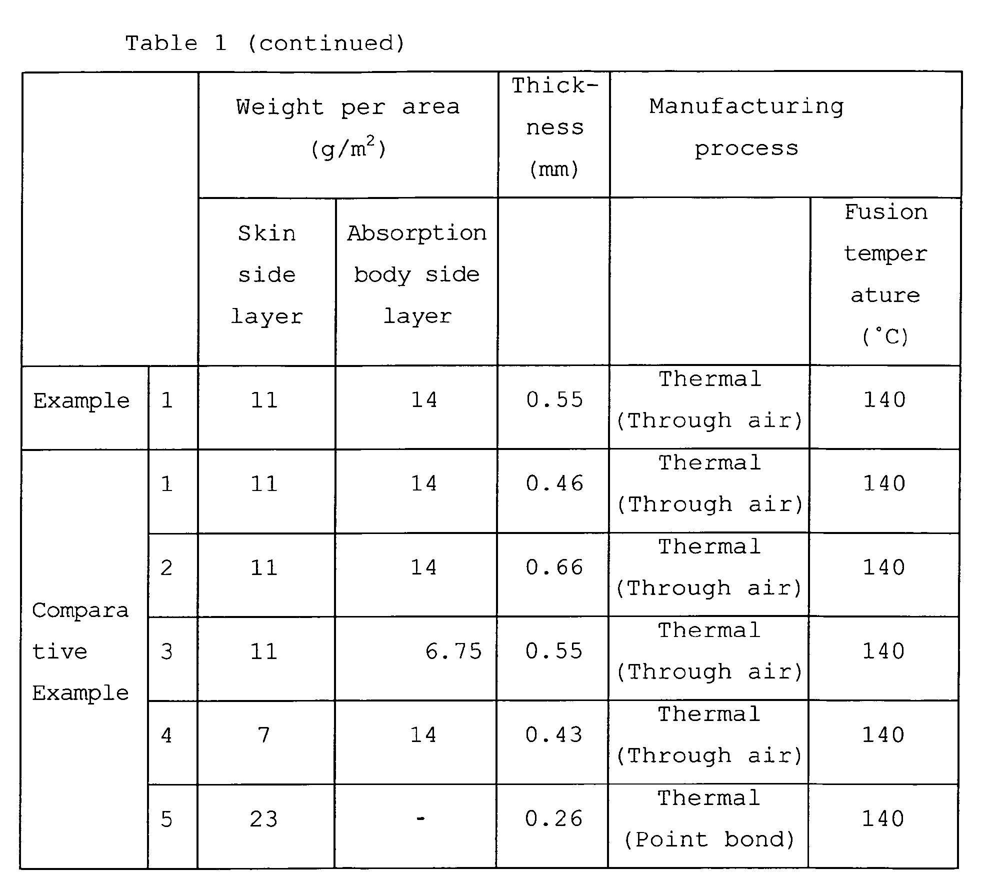

Next, sensory evaluations of the topsheets of Example and Comparative Examples manufactured in this way were performed. The sensory evaluation was performed as follows. The topsheet (6.5 cm x 6.5 cm) was overlaid with the side in contact with the skin up, on a dummy absorption body where 10 sheets of filter papers (corresponding to the No.2 grade filter paper in JIS P3801) having a size of 10 cm x 10 cm were overlapped. In that state, 0.085 g/cm² of saline (0.9% NaCl) at 20°C was dripped evenly on the topsheet. By touching the side in contact with the skin of the above topsheet at one minute after dripping, dry feeling and cold feeling were rated on a scale of one to seven as described below, and the results were shown in Table 2. Numeric values in Table 2 are average values obtained when five women touched the topsheet.

Dry feeling
7. Extremely dry
6. Dry
5. Slightly dry
4. Unconsciously dry
3. Slightly wet
2. Wet
1. Extremely wet

Cold feeling
7. Extremely warm
6. Warm
5. Slightly warm
4. Unconsciously cold
3. Slightly cold
2. Cold
1. Extremely cold

**Table 2**

| | | Contact warm/cold feeling (q-max value) | | Dry feeling | Cold feeling |
|---|---|---|---|---|---|
| | | Skin-side layer | Absorption-body side layer | | |
| Example | 1 | 102 | 186 | 6.4 | 4.2 |
| Comparative Example | 1 | 104 | 146 | 5.8 | 3.8 |
| | 2 | 137 | 104 | 3.0 | 2.6 |
| | 3 | 155 | 112 | 3.2 | 3.0 |
| | 4 | 133 | 184 | 3.0 | 3.0 |
| | 5 | 180 | 187 | 2.0 | 2.0 |

From these results, it is shown that in Example 1 where the q-max value during the wet condition on the face in contact with the skin is 1.1 or less and the difference between the face in contact with the skin and the face in contact with the absorption body is 0.5 or more, the dry feeling and the cold feeling are superior, whereas in Comparative Examples 1 to 5 where those are not satisfied, the dry feeling and the cold feeling are inferior.

Furthermore, the sensory evaluation was performed when the absorbent articles using the topsheets of Example and Comparative Examples were actually worn.

A pants type diaper was made as the absorbent articles by combining a liquid impermeable backsheet made up of polyethylene film and an absorbent core with the topsheet of Example 1 or Comparative Example 3. Seven healthy adult males and females wore these pants type diapers, and sat at rest in a room holding constant temperature and humidity at 26°C and 60% RH respectively, and 80 ml of saline (0.9% NaCl) at 37°C was injected by a silicone tube, and after 10 minutes, the sensory evaluation was performed on the following scale of one to five.

In Table 3, average values of the sensory evaluations by seven persons are shown. Here, the dry feeling was rated on the following scale of one to five.

Dry feeling
5. Extremely dry
4. Slightly dry
3 Neither dry nor wet
2. Slightly wet
1. Extremely wet

**Table 3**

| | Dry feeling |
|---|---|
| Example 1 | 2.48 |
| Comparative Example 3 | 2.00 |

When the results in Table 3 are confronted with the results in Tables 1 and 2, it is shown that in the cases (Example 1) of using the topsheets where the q-max value is within the scope of the present invention, the dry feeling is also high in the absorbent articles.

As discussed above, it has been shown that the dry feeling of the topsheet is excellent when the q-max value during the wet condition is 1.1 kw/m² or less on the face at the side in contact with the skin of the wearer and the q-max value on the face at the side in contact with the absorption body is 0.5 kw/m² or more larger than the q-max value on the face at the side in contact with the skin of the wearer. Conversely, this indicates that the topsheet with favorable dry feeling can be selected/evaluated by using the q-max value during the wet condition as an index, in more detail, using as a criterion that the q-max value during the wet condition is 1.1 kw/m² or less on the face at the side in contact with the skin of the wearer and the q-max value on the face at the side in contact with the absorption body is 0.5 kw/m² or more larger than the q-max value on the face at the side in contact with the skin of the wearer.

In the above Example and Comparative Examples, an example where the q-max value on the face in contact with the skin is reduced by giving the difference between the fineness of the face in contact with the skin and the face in contact with the absorption body of the topsheet and q-max value on the opposite side face large was cited. Also in the present invention, by giving the gradient to a fiber density of a fiber layer which constitutes the topsheet, it is possible to reduce the q-max value on the face in contact with the skin and further make the difference from the q-max value on the opposite side face large.

For example, the gradient of the fiber density can be given by giving the gradient to a thickness of each fiber layer which constitutes the topsheet. That is, when the fiber layer with a constant fiber density is made and the fiber layer varies in thickness, a section where the thickness of the fiber layer is large becomes the section where the fiber density is small. In the topsheet, when the thickness at the side in contact with the skin is represented by a, the thickness at the absorption body side is represented by b, and a and b satisfy a relation of a > b, it is possible to reduce the q-max value on the face in contact with the skin. In addition, when the difference of a > b is larger, the larger gradient occurs, and it is preferable. As a result, it is possible to increase the difference between the q-max value on the face at the absorption body side and the q-max value on the face at the side in contact with the skin, and it is possible to make the dry feeling more favorable.

Furthermore, the example of using thermal bond with the through air was cited in Example, but the similar advantage can be obtained by giving the gradient to the thickness of point bond. In order to give the gradient to the thickness of point bond, there are a method where heat crimping fibers are used, and the fibers are heat-crimped by heat-fusing by heat emboss, followed by heating again, or a method of widening pitches of dots of emboss.

As described above, according to the present invention, it is possible to obtain the topsheet of the absorbent article with favorable dry feeling, and select and/or evaluate the topsheet with favorable dry feeling. Therefore, the present invention can provide the topsheet meeting the needs of consumers by applying it in the production site for the topsheet of the absorbent article.

## Claims

1. A topsheet with liquid permeability of an absorbent article **characterized in that** a maximum heat transfer quantity (q-max value) during a wet condition that corresponds to a contact warm/cool feeling is 1.1 kw/m² or less on a face at a side in contact with skin of a wearer of the topsheet and a q-max value on a face at a side in contact with an absorption body is 0.5 kw/m² or more larger than the q-max value on the face at the side in contact with the skin of the wearer.

2. The topsheet according to claim 1, wherein a fiber density of a fiber layer that constitutes the topsheet is made higher in the face at the side in contact with the skin of the wearer than in the face at the side in contact with the absorption body.

3. The topsheet according to claim 1 or 2, wherein a fineness of a fiber layer that constitutes the topsheet is made lower in the face at the side in contact with the skin of the wearer than in the face at the side in contact with the absorption body.

4. An absorbent article comprising the topsheet according to any one of claims 1 to 3, a liquid impermeable backsheet, and an absorbent core disposed between the topsheet and the backsheet.

5. A method for selecting and/or evaluating a topsheet of an absorbent article with a favorable dry feeling comprising using a maximum heat transfer quantity (q-max value) during a wet condition measured as a contact warm/cool feeling as an index.

6. A method for selecting and/or evaluating a topsheet of an absorbent article with a favorable dry feeling comprising using as a criterion that a maximum heat transfer quantity (q-max value) during a wet condition measured as a contact warm/cool feeling is 1.1 kw/m² or less on a face at a side in contact with skin of a wearer of the topsheet and a q-max value on a face at a side in contact with an absorption body is 0.5 kw/m² or more larger than the q-max value on the face at the side in contact with the skin of the wearer.
